# EUROPÄISCHE PATENTANMELDUNG

(11) **EP 1 498 424 A2**
(43) Veröffentlichungstag der Anmeldung: **19.01.2005**
(21) Anmeldenummer: 04090140.7
(22) Anmeldetag: 08.04.2004
(51) Int. Cl.: C07K 14/47, G01N 33/574, C12Q 1/68, C07K 16/18

(54) **Humane Nukleinsäuresequenzen aus Bronchialkarzinomen**

(30) Priorität: 09.04.2003 DE 10316701
(71) Anmelder: Hinzmann, Bernd, Dr., 13127 Berlin (DE); Hermann, Klaus Dr., 12679 Berlin (DE); Heiden, Esmeralda, 10589 Berlin (DE); Rosenthal, André, Prof., 14482 Potsdam (DE)
(72) Erfinder: Rosenthal, André, Prof. Dr., 14482 Potsdam (DE); Hermann, Klaus, Dr., 12679 Berlin (DE); Heiden, Esmeralda, Dr., 10589 Berlin (DE); Pilarsky, Christian, Dr., 01309 Dresden (DE); Brümmendorf, Thomas, Dr., 79540 Lörrach (DE); Staub, Eike, 13189 Berlin (DE); Röpcke, Stefan, 12163 Berlin (DE); Mennerich, Detlev, Dr., 10551 Berlin (DE); Kinnemann, Henrik, Dr., 10589 Berlin (DE); Li, Xinzhong, Dr., c/o Dept. of Computing, London SW7 2BZ (GB)

(57) **Zusammenfassung**

Die Erfindung betrifft neue humane Nukleinsäuresequenzen aus Bronchialkarzinomen, hierdurch codierte Proteine bzw. Peptide sowie deren Verwendungen im Zusammenhang mit der Diagnose und/oder Behandlung von Bronchialkrebs.

## Beschreibung

### Gebiet der Erfindung

Die Erfindung betrifft neue humane Nukleinsäuresequenzen aus Bronchialkarzinomen sowie hierdurch codierte Proteine bzw. Peptide, die Verwendung von hieraus abgeleiteten Sequenzen zum Screenen nach daran bindenden Substanzen, sowie die Verwendung von an solche Nukleinsäuresequenzen und Proteine bzw. Peptide bindenden Substanzen zur Diagnose und/oder Behandlung von Tumorerkrankungen, insbesondere Bronchialkrebs.

### Hintergrund der Erfindung und Stand der Technik

Krebs, insbesondere Lungenkrebs, ist eine mit zunehmendem Alter und im Zusammenhang mit Nikotinkonsum mit beachtlicher Inzidenz auftretende Erkrankung. Das Bronchialkarzinom ist derzeit die häufigste Todesursache aller Krebskranken Männer und Frauen in Nordamerika und Europa. Die Fünf-Jahres-Überlebensrate aller Bronchialtumorpatienten liegt bei nur 13%. Lediglich die Patienten mit noch lokal begrenzten Lungentumorerkrankungen zeigen eine Fünf-Jahres-Überlebensrate von 46%. Allerdings werden nur 16% der Bronchialkarzinome in diesem frühen Tumorstadium diagnostiziert.

Bislang wird die Lungentumorerkrankung im wesentlichen durch eine histopathologische Diagnose manifestiert. Die anschließende Therapie richtet sich nach dem Tumorstadium, der Lokalisation und der Ausbreitung des Tumors sowie nach der körperlichen Verfassung des Patienten. Die Therapie der Wahl bei einem nicht-kleinzelligen Lungenkarzinom der frühen Stadien besteht in der potentiell kurativen Operation. Dies bezieht sich auf die Stadien I und II und in Verbindung mit postoperativer Strahlentherapie auch auf das Stadium IIIA. Eine primäre Operation ist jedoch nur bei 25-30% aller Patienten möglich. Im Stadium IIIA mit Lymphknotenmetastasen sowie im Stadium IIIB dominiert die Radiotherapie. In den letzten Jahren tritt bei diesen Patienten der multimodale Therapieansatz mehr und mehr in den Vordergrund. Bei gering ausgeprägtem und vermutlich technisch vollständig resektablem N2 konkurrieren primäre Operation und Nachbestrahlung mit einem neoadjuvanten Therapieansatz und anschließender Operation. Das neoadjuvante Konzept mit initialer Chemotherapie oder Chemoradiotherapie sowie anschließender Operation hat sich in der fortgeschrittenen N2 Situation bzw. im Stadium IIIB weitgehend durchgesetzt. Im disseminierten Stadium IV dominiert die Chemotherapie als palliative Behandlung, zum Teil ergänzt durch die Radiotherapie.

Der operative Eingriff mit einer Teilentfernung einzelner befallener Lungenlappen und anschließender Chemo- und/oder Radiotherapie hat verschiedene nachteilige Effekte auf die Physis und Psyche der betroffenen Patienten, was in der Regel zu einer starken Einschränkung der Lebensqualität führt. Daher ist eine verbesserte Diagnose und Behandlung von Bronchialtumorpatienten, insbesondere mit einer Verbesserung der Überlebenszeiten und Lebensqualität, in einem hohem Maße wünschenswert.

### Technisches Problem der Erfindung

Der Erfindung liegt daher das technische Problem zugrunde, pharmazeutische Zusammensetzungen zur Diagnose und/oder zur Behandlung von Bronchialkrebs-erkrankungen anzugeben sowie Mittel zu deren Findung.

Grundzüge der Erfindung sowie bevorzugte Ausführungsbeispiele.

Zur Lösung dieses technischen Problems lehrt die Erfindung zunächst eine Nukleinsäure enthaltend oder bestehend aus einer Nukleinsäuresequenz gemäß einer der Sequenzen Seq.-ID 1 bis 489 sowie ein Peptid oder Protein enthaltend eine Aminosäurensequenz codiert durch eine der Nukleinsäuresequenzen Seq.-ID 1 bis 489 oder bestehend hieraus oder enthaltend eine Aminosäurensequenz gemäß einer der Sequenzen 499 bis 978 oder bestehend hieraus. Erfindungsgemäße Nukleinsäuren oder Proteine bzw. Peptide lassen sich mit den üblichen molekularbiologischen Methoden herstellen.

Die Erfindung betrifft weiterhin verschiedene Verwendungen der neuen Nukleinsäuren bzw. Peptide oder Protein, ebenso wie (gleiche) Verwendungen bereits bekannter Nukleinsäuren. Diese sind:
i) Verwendung einer erfindungsgemäßen Nukleinsäure und/oder eines erfindungsgemäßen Peptids oder Proteins, zur Detektion von Bronchialkrebs oder zur Detektion eines Risikos der Erkrankung an Bronchialkrebs, wobei eine Lungen- bzw. Bronchial-Gewebeprobe auf Übertranskription der Nukleinsäure oder auf Überexpression des Proteins untersucht wird. Dabei kann eine an die Nukleinsäure oder eine an das Protein oder Peptid bindende Detektorsubstanz, vorzugsweise enthaltend eine Reportergruppe, verwendet werden, wobei Bindung besagter Nukleinsäure und/oder besagten Proteins oder Peptids an die Detektorsubstanz halbquantitativ oder quantitativ detektiert wird. Auch kann das Expressionsniveau durch Amplifikation, beispielsweise quantitative PCR, gemessen werden.
ii) Verwendung einer erfindungsgemäßen Nukleinsäure oder eines erfindungsgemäßen Proteins oder Peptids zum Screenen nach daran bindenden Substanzen, insbesondere prospektiven Wirkstoffen zur Inhibierung von besagter Nukleinsäure oder besagtem Protein oder Peptid, oder prospektiven Detektorsubstanzen, wobei eine prospektive Substanz oder eine Mischung solcher prospektiver Substanzen mit besagter Nukleinsäure oder besagtem Protein oder Peptid kontaktiert wird, wobei mit einem Bindungsassay Bindungsereignisse festgestellt werden, und wobei eine bindende prospektive Substanz, ggf. nach Dekonvolution, selektiert wird.
iii) Verwendung einer eine erfindungsgemäße Nukleinsäure oder ein erfindungsgemäßes Peptid bzw. Protein inhibierenden oder daran bindenden Substanz, insbesondere identifiziert mit dem erfindungsgemäßen Screening Verfahren, zur Herstellung einer pharmazeutischen Zusammensetzung zur Diagnose und/oder Behandlung von Bronchialkrebs.

Eine im Rahmen der Erfindung eingesetzte Substanz kann ausgewählt sein aus der Gruppe bestehend aus:
a) Antisense-Oligonukleotide, siRNA, und Ribozyme gegen eine Nukleinsäure nach Anspruch 1,
b) an ein Peptid oder Protein nach Anspruch 2 bindendes, insbesondere nach Anspruch 5 identifiziertes, organisches Molekül mit einem Molekulargewicht unterhalb 5000, vorzugsweise unterhalb 1000, höchstvorzugsweise unterhalb 300,
c) Aptamer gegen ein Protein oder Peptid nach Anspruch 2, insbesondere identifiziert nach Anspruch 5,
d) (monoklonaler) Antikörper, insbesondere humaner oder humanisierter Antikörper gegen ein Protein oder Peptid nach Anspruch 2,
e) anti-idiotypischer nicht-humaner (monoklonale) Antikörper, generiert mittels eines Antikörpers der Unterguppe d), und
f) vorstehende Substanzen derivatisiert mit einer Reportergruppe, einem Zelltoxin, einer immunstimulierenden Komponente und/oder einem Radioisotop.

Im Falle a) kann als Ribozyme beispielsweise ein Hammerhead Ribozym eingesetzt werden. Die Ribozym-Schnittstelle wird mit der Maßgabe ausgewählt, dass durch die Aktivität des Ribozymes die Expression des Proteins entweder unterbunden wird, oder eine inaktive Form bzw. ein inaktives Fragment des Proteins exprimiert wird. Beides läßt sich beispielsweise dadurch ermitteln, dass in einem Zellsystem, in welchem ein erfindungsgemäßes Protein auf definiertem Niveau exprimiert wird, dieses Zellsystem mit einem oder mehreren für definierte Schnittstellen modelliertes Ribozym kontaktiert wird und das Expressionsniveau bestimmt bzw. die biologische Aktivität des exprimierten Proteins. Dies wird dann verglichen mit einer Negativprobe bzw. den Ergebnissen ohne Kontaktierung und Ribozyme werden selektiert, die zu niedrigerer Expression oder Aktivität führen. Entsprechend kann im Falle der siRNA oder der antisense Nukleinsäuren vorgegangen werden. Im Falle a) sind alle Sequenzen brauchbar.

Im Falle b) können chemische Stoffbibliotheken eingesetzt werden, um nach bindenden Substanzen zu screenen. Eine Validierung bindender Substanzen für therapeutische Zwecke kann durch Bestimmung der biologischen Aktivität des Proteins in einem Zellsystem mit und ohne Kontaktierung und Vergleich der erhaltenen Ergebnisse erfolgen. Für therapeutische Zwecke ausgewählt werden dann solche Stoffe, die zu einer reduzierten biologischen Aktivität führen. Es ist auch möglich, dass im Rahmen eines erfindungsgemäßen Screening Verfahren an Stelle der Bindung die biologische Aktivität bestimmt wird; dann ist eine Validierung im vorstehenden Sinne zugleich mit dem Screening erfolgt. Biologische Aktivität läßt sich beispielsweise dadurch bestimmen, dass natürliche Assoziationspartner des Proteins bestimmt und deren Vorkommen und Form (z.B. Monomer/Dimer/Heterodimer) untersucht werden. Es lassen sich auch weiter downstream in einer Stoffwechselkaskade entstehende Substanzen als Indikator verwenden; diese lassen sich beispielsweise dadurch identifizieren, dass zuvor Zellkomponenten analysiert werden für die das Protein exprimierende Zelle und ein Vergleich durchgeführt wird mit gleichen Zellen, in welchen jedoch die Expression gentechnisch deletiert ist. Im Einzelnen ist im Falle (b) folgendes anzumerken. Insbesondere ausgehend von den Nukleinsäuresequenzen 3; 6; 8; 9; 13; 15; 19; 20; ; 21; 23; 26; 28; 29; 30; 31; 32; 35; 37; 41; 46; 50; 52; 53; 55; 57; 58; 60; 62; 66; 68; 73; 77; 78; 79; 80; 81; 83; 87; 89; 92; 96; 99; 102; 112; 112.2; 112.3; 112.4; 112.5; 112.6; 124; 125; 129; 131; 135; 138; 139; 140; 142; 144; 146; 149; 154; 157; 160; 163; 165; 167; 168; 169; 170; 171; 172; 173; 174; 177; 178; 180; 181; 182; 183; 188; 189; 190; 191; 192; 193; 194; 195; 197; 198; 199; 201; 203; 206; 207; 211; 212; 213; 214; 215; 216; 218; 220; 223; 224; 230; 232; 233; 234; 235; 238; 239; 241; 242; 243; 244; 247; 249; 250; 254; 259; 262; 263; 266; 267; 268; 269; 274; 278; 280; 282; 283; 284; 286; 287; 288; 290; 293; 296; 298; 299; 308; 310; 312; 315; 316; 317; 320; 320.2; 322; 323; 324; 326; 328; 334; 338; 339; 341; 342; 343; 344; 345; 346; 348; 354; 356; 357; 359; 360; 367; 369; 370; 372; 376; 379; 380; 394; 395; 396; 397; 399; 402; 412; 417; 421; 425; 428; 429; 431; 432; 439; 448; 452; 456; 461; 462; 463; 468; 470; 471; 472; 473; 474; 475; 476; 478; 482; 484; 487; 489 können Mimikry-Verbindungen abgeleitet werden, welche in der Lage sind, spezifisch das funktionell aktive Zentrum der Enzymdomänen-enthaltenden Proteine zu binden und in ihrer biologischen Aktivität zu inhibieren. Sollte eine Mimikry-Verbindung in der Lage sein, spezifisch die Peptide 492; 495; 497; 498; 502; 504; 508; 509; 510; 512; 515; 517; 518; 519; 520; 521; 524; 526; 530; 535; 539; 541; 542; 544; 546; 547; 549; 551; 555; 557; 562; 566; 567; 568; 569; 570; 572; 576; 578; 581; 585; 588; 591; 601; 601.2; 601.3; 601.4; 601.5; 601.6; 613; 614; 618; 620; 624; 627; 628; 629; 631; 633; 635; 638; 643; 646; 649; 652; 654; 656; 657; 658; 659; 660; 661; 662; 663; 666; 667; 669; 670; 671; 672; 677; 678; 679; 680; 681; 682; 683; 684; 686; 687; 688; 690; 692; 695; 696; 700; 701; 702; 703; 704; 705; 707; 709; 712; 713; 719; 721; 722; 723; 724; 727; 728; 729; 730; 731; 732; 733; 736; 738; 739; 743; 748; 751; 752; 755; 756; 757; 758; 763; 767; 769; 771; 772; 773; 775; 776; 777; 779; 782; 785; 787; 788; 797; 799; 801; 804; 805; 806; 809; 809.2; 811; 812; 813; 815; 817; 823; 827; 828; 830; 831; 832; 833; 834; 835; 837; 843; 845; 846; 848; 849; 856; 858; 859; 861; 865; 868; 869; 883; 884; 885; 886; 888; 891; 901; 906; 910; 914; 917; 918; 920; 921; 928; 937; 941; 945; 950; 951; 952; 957; 959; 960; 961; 962; 963; 964; 965; 967; 971; 973; 976; 978 zu binden, ohne es in ihrer biologischen Funktion zu inhibieren, kann es trotzdem zur Lösung des technischen und medizinischen Problems beitragen. Es kann als guter Binder mit einem Zelltoxin derivatisiert werden, um somit in einem Targeting Approach die Zielzellen abzutöten. Ein solches Zelltoxin kann ein klassisches Chemotherapeutikum aber auch ein Radioisotop sein. Ebenfalls können solche Peptidspezifische Mimikry-Verbindungen, mit einer Reportergruppe derivatisiert, als Verbindung in der Tumordiagnostik verwendet werden.

Geeignete Aptamere (c) lassen sich unschwer beispielsweise mittels des wohlbekannten SELEX Verfahren identifizieren, wobei das erfindungsgemäße Protein als Target eingesetzt wird. Einsetzbar sind alle Sequenzen.

Antikörper (d), insbesondere monoklonale Antikörper, können in üblicher Weise durch Immunisierung eines nichtmenschlichen Säugetiers mit einem erfindungsgemäßen Protein, einer erfindungsgemäßen Nukleinsäure (z.B. cDNA), einer ein erfindungsgemäßes Protein konstitutiv exprimierenden Zelle (Krebszelle oder beispielsweise mit einer erfindungsgemäßen Nukleinsäure transfizierte Zelle, wie COS oder NIH3T3), oder mittels recombinant hergestelltem erfindungsgemäßem Protein oder Peptid, beispielsweise in E.coli oder Eukaryontenzellen (z.B. Insektenzellen) exprimiert, erhalten werden. Monoklonale Antikörper sind durch übliche Selektion und Etabilierung von Hybridomzellen erhältlich. Auch kann die Phage Display Technologie zur Generierung der Antikörper eingesetzt werden. Für den Fall (d) ist ergänzend folgendes anzumerken. Ausgehend insbesondere von extrazellulär exponierten Peptiden oder Proteinen 490; 491; 493; 494; 496; 499; 500; 501; 502; 503; 505; 506; 507; 511; 513; 514; 516; 520; 522; 523; 525; 527; 528; 529; 530; 531; 532; 533; 534; 536; 537; 538; 540; 543; 545; 548; 550; 551; 552; 553; 554; 556; 557; 558; 559; 560; 561; 563; 564; 565; 568; 571; 573; 574; 575; 577; 578; 579; 580; 582; 583; 584; 585; 586; 587; 589; 590; 592; 593; 594; 595; 596; 597; 598; 599; 600; 602; 603; 604; 605; 606; 607; 608; 609; 610; 611; 612; 615; 616; 617; 619; 621; 622; 623; 625; 626; 630; 631; 632; 634; 636; 637; 638; 639; 640; 641; 642; 644; 645; 647; 648; 650; 651; 653; 655; 658; 664; 665; 668; 669; 670; 673; 674; 675; 676; 678; 682; 683; 685; 689; 691; 693; 694; 697; 698; 699; 706; 707; 708; 710; 711; 712; 714; 715; 716; 717; 718; 720; 721; 725; 726; 734; 735; 737; 740; 741; 742; 744; 745; 746; 747; 749; 750; 753; 754; 757; 758; 759; 760; 761; 762; 764; 765; 766; 768; 770; 772; 774; 778; 780; 781; 782; 783; 784; 786; 788; 789; 790; 791; 792; 793; 794; 795; 796; 798; 800; 802; 803; 804; 805; 807; 808; 810; 813; 814; 816; 817; 818; 819; 820; 821; 822; 823; 824; 825; 826; 828; 829; 830; 836; 838; 839; 840; 841; 842; 843; 844; 847; 850; 851; 852; 853; 854; 855; 856; 857; 860; 862; 863; 864; 866; 867; 870; 871; 872; 873; 874; 875; 876; 877; 878; 879; 880; 881; 882; 884; 886; 887; 889; 890; 891; 892; 893; 894; 895; 896; 897; 898; 899; 900; 902; 903; 904; 905; 907; 908; 909; 910; 911; 912; 913; 914; 915; 916; 919; 922; 923; 924; 925; 926; 927; 929; 930; 931; 932; 933; 934; 935; 936; 937; 938; 939; 940; 942; 943; 944; 946; 947; 948; 949; 953; 954; 955; 956; 958; 959; 961; 962; 963; 966; 967; 968; 969; 970; 972; 974; 975; 977 kann ein Antikörper in humaner und/oder humanisierter Form abgeleitet werden, welcher in der Lage ist, spezifisch das funktionell aktive Zentrum der Proteine zu binden und in deren biologischer Aktivität zu inhibieren. Ebenfalls kann ein solcher Peptidespezifischer Antikörper, mit einer Reportergruppe derivatisiert, als Verbindung in der Tumordiagnostik verwendet werden.

Im Falle der anti-idiotypischen Antikörper (e) sind diese dadurch erzeugbar, dass mittels eines erfindungsgemäßen Antikörpers, welcher nicht notwendigerweise die biologische Aktivität des erfindungsgemäßen Proteins beeinflussen muss, in einem nicht-menschliche Säugetier ein zweiter anti-idiotypischer (monoklonaler) Antikörper generiert wird. Dieser anti-idiotypische Antikörper täuscht dann bei Applikation in humane Zellen dem humanen Immunsystem ein Bild des Zielmoleküls vor und wird aufgrund seiner nicht-humanisierten Form als körperfremdes Epitop erkannt. Der Mensch bildet folglich natürlicherweise Antikörper gegen den anti-idiotypischen Antikörper und somit auch gegen das Protein bzw. gegen das Protein exprimierende Zellen. Diese Variante der Erfindung ist ausschließlich für therapeutische Zwecke verwendbar. Ausgehend von Peptid- bzw. Proteinsequenzen 490; 491; 493; 494; 496; 499; 500; 501; 502; 503; 505; 506; 507; 511; 513; 514; 516; 520; 522; 523; 525; 527; 528; 529; 530; 531; 532; 533; 534; 536; 537; 538; 540; 543; 545; 548; 550; 551; 552; 553; 554; 556; 557; 558; 559; 560; 561; 563; 564; 565; 568; 571; 573; 574; 575; 577; 578; 579; 580; 582; 583; 584; 585; 586; 587; 589; 590; 592; 593; 594; 595; 596; 597; 598; 599; 600; 602; 603; 604; 605; 606; 607; 608; 609; 610; 611; 612; 615; 616; 617; 619; 621; 622; 623; 625; 626; 630; 631; 632; 634; 636; 637; 638; 639; 640; 641; 642; 644; 645; 647; 648; 650; 651; 653; 655; 658; 664; 665; 668; 669; 670; 673; 674; 675; 676; 678; 682; 683; 685; 689; 691; 693; 694; 697; 698; 699; 706; 707; 708; 710; 711; 712; 714; 715; 716; 717; 718; 720; 721; 725; 726; 734; 735; 737; 740; 741; 742; 744; 745; 746; 747; 749; 750; 753; 754; 757; 758; 759; 760; 761; 762; 764; 765; 766; 768; 770; 772; 774; 778; 780; 781; 782; 783; 784; 786; 788; 789; 790; 791; 792; 793; 794; 795; 796; 798; 800; 802; 803; 804; 805; 807; 808; 810; 813; 814; 816; 817; 818; 819; 820; 821; 822; 823; 824; 825; 826; 828; 829; 830; 836; 838; 839; 840; 841; 842; 843; 844; 847; 850; 851; 852; 853; 854; 855; 856; 857; 860; 862; 863; 864; 866; 867; 870; 871; 872; 873; 874; 875; 876; 877; 878; 879; 880; 881; 882; 884; 886; 887; 889; 890; 891; 892; 893; 894; 895; 896; 897; 898; 899; 900; 902; 903; 904; 905; 907; 908; 909; 910; 911; 912; 913; 914; 915; 916; 919; 922; 923; 924; 925; 926; 927; 929; 930; 931; 932; 933; 934; 935; 936; 937; 938; 939; 940; 942; 943; 944; 946; 947; 948; 949; 953; 954; 955; 956; 958; 959; 961; 962; 963; 966; 967; 968; 969; 970; 972; 974; 975; 977 können monoklonale Maus/Ratten Antikörper abgeleitet werden. Die monoklonalen Antikörper Ab1 müssen nicht notwendiger Weise in der Lage sein, spezifisch das funktionell aktive Zentrum der Proteine zu binden und in deren biologischer Aktivität zu inhibieren. Die monoklonalen Antikörper Ab1 werden im zweiten Schritt verwendet, um zweite anti-idiotypische maus-monoklonale Antikörper aAB1 zu generieren. Die monoklonalen aAB1 stellen die Verbindung zur Lösung des technischen und medizinischen Problems dar, in dem diese dem humanen Immunsystem ein Image des Antigens "vertäuscht" und durch seine nicht-humanisierte Form als körperfremdes Epitop erkannt wird. Der humane Körper bildet zwangsläufig eigene Antikörper gegen aAB1 und somit gegen die das Zielprotein exprimierenden Tumorzellen. Während sich die aAB1 Antikörper aussschliesslich zur Behandlung der Tumorerkrankung eignen, sind die Ab1 Antikörper ausschliesslich für die diagnostische Problemlösung zu verwenden.

Im Falle f) können ausgehend von den Nukleinsäuresequenzen 1 bis 489 und/oder den Aminosäuresequenzen 490 bis 978 Antikörper und/oder Mimikry-Verbindungen mit hoher spezifischen Bindungsaktivität isoliert werden. Solche Substanzen werden dann in einem zweiten Schritt mit Zelltoxinen und/oder Radioisotopen derivatisiert und zum Targeting der Tumorzellen eingesetzt.

Die Erfindung betrifft des weiteren ein Verfahren zur Diagnose einer Bronchialkrebserkrankung, wobei eine erfindungsgemäße pharmazeutische Zusammensetzung in der Ausführungsform mit einer Reportergruppe in zu untersuchendes Gewebe in vivo oder in vitro appliziert wird, wobei das zu untersuchende Gewebe dann einer Detektionsverfahrenstufe unterworfen wird, welche sensitiv für die Reportergruppe ist, und wobei im Fall der Detektion eines definierten Mindestwertes der Reportergruppe im Gewebe das Gewebe als Tumorzellen enthaltend qualifiziert wird, sowie ein Verfahren zur Behandlung einer Bronchialkrebs-erkrankung, wobei eine erfindungsgemäße pharmazeutische Zusammensetzung in einer physiologisch wirksamen Dosis einem Patienten dargereicht wird.

Die Erfindung beruht auf der Erkenntnis, daß erfindungsgemäße Gene bzw. Genprodukte differentiell in Bronchialtumorgewebe exprimiert werden, i.e. in Bronchialtumorgewebe ist die Expression höher oder niedriger, insbesondere höher, verglichen mit normalen Zellen gleichen Gewebes. Dies erlaubt es einerseits, insbesondere diese neuen Gene bzw. Genprodukte als Marker zur Identifizierung von Tumorzellen im Bronchialgewebe zu nutzen. Dabei kann die Untersuchung auf Marker in Proben von Blutserum oder Sputum erfolgen, was insbesondere im Bereich der Vorsorgeuntersuchung bzw. Frühdiagnostik vorteilhaft ist. Auf der anderen Seite bietet die Inhibierung der Gene bzw. Genprodukte, insbesondere auch bei lokaler Applikation, die Möglichkeit, in die Bronchialtumor-spezifischen Genprodukt-Assoziationen mit anderen Prozessen in den Tumorzellen einzugreifen und somit letztendlich den tumorzellenspezifisch veränderten Stoffwechsel zu stören und zu einem Absterben oder zumindest einer Wachstumshemmung der Bronchialtumorzellen beizutragen.

Im Rahmen der Erfindung kann es sich empfehlen, im Vorfeld einer Behandlung mit einer erfindungsgemäßen pharmazeutischen Zusammensetzung eine Probe aus einem Gewebe, welches als Tumorgewebe mit anderen Methoden identifiziert ist, zu entnehmen und die Gewebeprobe auf Expression bzw. Überexpression des erfindungsgemäßen Gens bzw. Genproduktes zu untersuchen. Alternativ kann mit einer erfindungsgemäßen Detektorsubstanz zur Diagnose in vivo auf Abhängigkeit von dem Gen bzw. Genprodukt getestet werden. Wird eine Expression bzw. Überexpression des Gens bzw. Genproduktes gegenüber Normalgewebe gleichen Typs festgestellt, so ist die Anwendung der erfindungsgemäßen pharmazeutischen Zusammensetzung indiziert.

Handelt es sich bei dem Tumor um einem Typus, bei welchem Tumorzellen ein erfindungsgemäßes Gen exprimieren, Normalzellen gleichen Gewebetyps jedoch nicht oder nur schwach, so ist es besonders bevorzugt, wenn die an das Gen bzw. das Genprodukt bindende Substanz zusätzlich eine zytotoxische und/oder immunstimulierende Komponente trägt. Dies führt dann letztendlich dazu, dass praktisch ausschließlich Tumorzellen getötet werden, sei es durch die Zytotoxizität, sei es durch Angriff durch das stimulierte Immunsystem, während Normalzellen in dem Gewebe praktisch vollständig erhalten bleiben. In dieser Ausführungsform braucht die bindende Substanz selbst nicht inhibierend auf das Gen bzw. Genprodukt zu wirken, da die bindende Substanz dann lediglich als Marker funktionieren muß, welcher die Komponenten zu Ziel-Tumorzellen trägt. Im Falle des Einsatzes einer zytotoxischen und/oder immunstimulierenden Komponente kann es sich besonders empfehlen, wenn die pharmazeutische Zusammensetzung zur lokalen Applikation in Tumorzellen enthaltendem Gewebe hergerichtet ist, beispielsweise zur Injektion.

Sofern im Rahmen der Beschreibung offenbarte und/oder beanspruchte Sequenzen per se vorbekannt sind oder Teile vorbekannter Sequenzen sind, sind die offenbarten Sequenzen, soweit sie mit vorbekannten Sequenzen übereinstimmen, insofern Gegenstand der Erfindung, als dass sie lediglich gemäß den beschriebenen Verwendungen eingesetzt werden. Offenbarte und/oder beanspruchte Sequenzen, welche Teile von vorbekannten Sequenzen sind, können mittels eines Disclaimers oder mehrerer Disclaimer in Ansprüchen so abgegrenzt werden, dass die vorbekannten Sequenzen nicht mit umfasst sind.

Die Nukleinsäurensequenzen 1 bis 171 sowie die Peptidsequenzen 490 bis 660 sind insbesondere geeignet im Falle von Adenokarzinomen. Die Nukleinsäuresequenzen 172 bis 290 und die Peptidsequenzen 661 bis 779 sind insbesondere geeignet im Falle von Adenokarzinomen und Plattenepithelkarzinomen bzw. den nicht-kleinzelligen Bronchialkarzinomen. Die Nukleinsäuresequenzen 291 bis 489 sowie die Peptidsequenzen 780 bis 978 sind insbesondere geeignet im Falle von Plattenepithelkarzinomen.

### Definitionen.

Im Rahmen dieser Beschreibung umfasst eine Sequenz alle humanen Isoformen, Splice Varianten und regulatorische RNA, bekannt oder neu, auf Nukleinsäuren- oder Aminosäurenbasis, verwendet. Mit diesen Begriffen mit umfaßt sind auch die ggf. im Rahmen dieser Beschreibung offenbarten kurzen Sequenzen, welche aus den verschiedenen Varianten stammen, beispielsweise Immunisierungssequenzen. Weiterhin mit umfaßt sind auch Homologe, wobei die Homologie zumindest 80%, vorzugsweise mehr als 90%, höchstvorzugsweise mehr als 95%, beträgt (berechnet mit dem Programm MEGALIGN, DNASTAR LASERGENE, in der zum nmeldezeitpunkt gültigen Fassung). Im Falle der Nukleinsäuresequenzen sind auch komplementäre oder allelische Varianten mit umfaßt. Weiterhin sind Sequenzen umfaßt, welche lediglich Teilsequenzen, beispielsweise ein Exon oder mehrere Exons, der explizit offenbarten Sequenzen oder komplementärer Sequenzen hierzu darstellen, mit der Maßgabe, daß diese Teilsequenzen im Falle der Nukleinsäuren eine für eine Hybridisierung mit einer erfindungsgemäßen Nukleinsäure hinreichende Länge, zumindest 50 oder 150 Basen, aufweisen und im Falle der Proteine bzw. Peptide, ggf. codiert durch eine Nukleinsäure, mit zumindest gleicher Affinität an ein protein- oder peptidspezifisches Zielmoleküle binden. Weiterhin sind alle mit erfindungsgemäß eingesetzten Nukleinsäuren hybridisierende Nukleinsäuren umfasst, nämlich solche, die unter stringenten Bedingungen (5°C bis 25°C unterhalb der Aufschmelztemperatur; siehe ergänzend J.M. Sambrook et al., A laboratory manual, Cold Spring Harbor Laboratory Press (1989) und E.M. Southern, J Mol Biol, 98:503ff (1975)) hybridisieren. Es versteht sich, daß die Erfindung auch Expressionskassetten umfaßt, i.e. eine oder mehrere der erfindungsgemäßen Nukleinsäuresequenzen mit mindestens einer Kontroll- oder regulatorischen Sequenz. Eine solche Expressionskassette kann auch eine Sequenz für ein bekanntes Protein umfassen, wobei im Zuge der Translation ein Fusionsprotein aus einem bekannten Protein und einem erfindungsgemäßen Protein oder Peptid entsteht. Ebenso sind auch antisense Sequenzen zu den vorstehenden Nukleinsäuresequenzen umfaßt. Mit umfaßt sind auch Expressionsvektoren enthaltend erfindungsgemäße Nukleinsäuren sowie damit transformierte Wirtszellen. Schließlich sind RNA sowie damit korrelierende DNA und umgekehrt umfaßt, ebenso wie genomische DNA als auch korrelierte cDNA und umgekehrt.

Im Zusammenhang mit erfindungsgemäßen Verwendungen umfassen die Begriffe der Nukleinsäuren oder Proteine bzw. Peptide neben den Volllängen der offenbarten Sequenzen (siehe auch vorstehender Absatz) auch Teilsequenzen hieraus, und zwar mit einer Mindestlänge von 12 Nukleotiden, vorzugsweise einer Mindestlänge von 30 bis 90 Nukleotiden, im Falle der Nukleisäuren und einer Mindestlänge von 4 Aminosäuren, vorzugsweise einer Mindestlänge von 10 bis 30 Aminosäuren, im Falle der Proteine oder Peptide. Insbesondere zwischen 12 und 90 sowie 4 und 30 ist jeder einzelne Zahlenwert als Mindestlänge möglich.

Die Begriffe der Detektion und/oder der Behandlung umfassen optional auch die Detektion und/oder Behandlung von Metastasen aus Primärtumoren in sonstigen Geweben. Der Begriff der Behandlung umfasst auch die Prophylaxe sowie die Nachbehandlung (bei nicht mehr detektierbarem Tumor oder bei stabilem Tumor). Der Begriff der Prophylaxe umfasst im Zusammenhang mit der Detektion auch die Vorsorgeuntersuchung.

Als Inhibitor ist eine Verbindung oder Substanz bezeichnet, welche entweder die Bildung von des Targetmoleküls inhibiert oder gebildetes Targetmolekül in der Aktivität reduziert, bezogen auf die in vitro oder in vivo Aktivität in Abwesenheit des Inhibitors. Insofern kann ein Inhibitor einerseits eine Substanz sein, welche in der Entstehungskaskade des Targetmoleküls inhibierend eingreift. Auf der anderen Seite kann ein Inhibitor eine Substanz sein, welche mit gebildetem Targetmolekül eine Bindung eingeht, und zwar dergestalt, dass weitere physiologische Wechselwirkungen mit endogenen Substanzen zumindest reduziert sind. Als Inhibitoren können auch Moleküle dienen, die die Transkription des Targetmoleküls beeinflussen und inhibieren. Solche Moleküle können Polyamide oder Zinkfingerproteine sein, die durch Bindung an DNA-Regionen der basalen Transkriptionsmaschinerie die Transkription verhindern. Die Transkription kann indirekt auch über die Inhibierung von Transkriptionsfaktoren geschehen, die für die Transkription des Zielgens essentiell sind. Die Inhibierung solcher Transkriptionsfaktoren kann über die Bindung an sogenannte Decoy-Aptamere gewährleistet werden.

Von der Erfindung mit umfasst sind Mimikry-Moleküle. Dies sind Verbindungen, die den variablen Bereich, insbesondere den Bindungsbereich eines Antikörpers nachbilden und an gleicher Stelle eines Zielmoleküls binden wie der zu Grunde liegende Antikörper. Desweiteren fallen Anticaline und Affibodies unter den Begriff der Mimikrimoleküle.

Der Begriff der Antikörper umfaßt humane, humanisierte und nicht-humanisierte polyklonale oder monoklonale Antikörper (typischerweise in vivo hergestellt). Der Begriff umfasst desweiteren Phage-Display-Antikörper, Ribozym-Display Antikörper (Bindung des Genotyps (mRNA) und des Phänotyps (protein) an Ribosomen) und RNA-Display Antikörper (in vitro hergestellt). Der Begriff umfasst auch Antikörper, welche durch Chimerisierung, Humanisierung oder De-Immunisierung (Ausschneiden von T-Zellepitopen aus dem humanen Antikörper, die unerwünschte Immunreaktionen auslösen) modifiziert sind, sowie spezifische Fragmente der leichten und/oder der schweren Kette des variablen Bereiches zu Grunde liegender Antikörper vorstehender Art. Die Herstellung bzw. Gewinnung solcher Antikörper mit vorgegebenen Immunogenen ist dem Durchschnittsfachmann wohl vertraut und braucht nicht näher erläutert zu werden. Weiterhin umfaßt sind bispezifische Antikörper, welche einerseits an ein Auslösemolekül einer Immun-Effektorzelle (z.B. CD3, CD16, CD64) und andererseits an ein Antigen der Tumorzielzelle binden. Dies bewirkt letzendlich im Bindungsfall, daß die Tumorzelle getötet wird.

Die galenische Herrichtung einer erfindungsgemäßen pharmazeutischen Zusammensetzung kann in fachüblicher Weise erfolgen. Als Gegenionen für ionische Verbindungen kommen beispielsweise Na⁺, K⁺, Li⁺ oder Cyclohexylammonium infrage. Geeigente feste oder flüssige galenische Zubereitungsformen sind beispielsweise Granulate, Pulver, Dragees, Tabletten, (Mikro-) Kapseln, Suppositorien, Sirupe, Säfte, Suspensionen, Emulsionen, Tropfen oder Lösungen zur Injektion (i.v., i.p., i.m., s.c.) oder Vernebelung (Aerosole), transdermale Systeme, sowie Präparate mit protrahierter Wirkstoff-Freigabe, bei deren Herstellung übliche Hilfsmittel wie Trägerstoffe, Spreng-, Binde-, Überzugs-, Quellungs-, Gleit- oder Schmiermittel, Geschmacksstoffe, Süßungsmittel und Lösungsvermittler, Verwendung finden. Als Hilfsstoffe sei Magnesiumcarbonat, Titandioxid, Lactose, Mannit und andere Zucker, Talcum, Milcheiweiß, Gelatine, Stärke, Zellulose und ihre Derivate, tierische und pflanzliche Öle wie Lebertran, Sonnenblumen-, Erdnuss- oder Sesamöl, Polyethylenglycole und Lösungsmittel, wie etwa steriles Wasser und ein- oder mehrwertige Alkohole, beispielsweise Glycerin, genannt. Eine erfindungsgemäße pharmazeutische Zusammensetzung ist dadurch herstellbar, dass mindestens ein erfindungsgemäß verwendeter Inhibitor in definierter Dosis mit einem pharmazeutisch geeigneten und physiologisch verträglichen Träger und ggf. weiteren geeigneten Wirk-, Zusatz- oder Hilfsstoffen mit definierter Inhibitordosis gemischt und zu der gewünschten Darreichungsform hergerichtet ist.

Tumorzellen exprimieren ein Protein differenziell, wenn Normalzellen des gleichen Gewebetyps dieses nicht oder nur in sehr geringen Mengen exprimieren. Tumorzellen überexprimieren ein Protein spezifisch bzw. differenziell, wenn dieses im Vergleich zu Normalzellen des gleichen Gewebes zumindest in doppelter Menge exprimiert wird. Es gilt Entsprechendes umgekehrt, wenn das Protein im Gegensatz zu Tumorzellen in normalen Zellen exprimiert bzw. überexprimiert wird.

Zytotoxische Komponenten bzw. Gruppen sind Verbindungen, welche direkt oder indirekt Apoptose und/oder Nekrose einleiten oder zumindest wachstumshemmend wirken. Solche Gruppen bzw. Verbindungen können neben Radioisotopen (z.B. 188Re, 213Bi, 99mTc, 90Y, 131J, 177Lu) insbesondere Toxine (z.B. Diphterie-, Pseudomonas-Toxin) oder Zytostatika einschließlich sogenannter Prodrugs sein, welche in der Tumortherapie eingesetzt werden. Beispiele hierfür sind: Alkylantien (z.B. Mechlorethamin, Ifosfamid, Chlorambucil, Cyclophosphamid, Melphalan, Alkylsulfonate, Busulphan, Nitrosoharnstoffe, Carmustin, Lomustin, Semustin, Triazene, Dacarbazin), Antimetaboliten (z.B. Folsäure-Antagonisten, Methotrexat, Pyrimidin-Analoga, Fluoruracil, Fluordesoxyuridin, Cytarabin, Gemcitabin, Purin-Analoga, Mercaptopurin), Mitosehemmer (z.B. Vincaalkaloide, Vincristin, Vinblastin, Paclitaxal, Docetaxel, Protaxel), Epipodophyllotoxine (z.B. Etoposid, Teniposid), Antibiotika (z.B. Dactinomycin, Daunorubicin, Idarubicin, Anthracycline, Bleomycin, L-Asparaginase), Platinkomplexverbindungen (z.B. Cisplatin), Hormone und verwandte Verbindungen (z.B. Nebennierensindensteroide, Aminogluthetimid, Gestagene, Östrogene, Androgene, Antiöstrogene, Tamoxifen, Steriodanaloga, Flutamid). Bei Bindung einer solchen Verbindung mit einer an das Protein bindenden Substanz erfolgt die Kopplung dergestalt, daß die Affinität zum Protein um nicht mehr als 90%, vorzugsweise 50%, bezogen auf die Substanz ohne zytostatische Gruppe, reduziert ist und die zytostatische Wirkung der Gruppe um nicht mehr als 90%, vorzugsweise 50%, bezogen auf die Verbindung ohne Substanz, reduziert ist.

Eine immunstimulierende Komponente ist meist ein Protein oder ein wirksamer Bestandteil hiervon, welches Zellen des Immunsystems stimuliert. Beispiele hierfür sind: Zytokine, wie M-CSF, GM-CSF, G-CSF, Interferone, wie IFN-alpha, beta, gamma, Interleukine wie IL-1 bis -16 (außer -8), human LIF, Chemokine wie Rantes, MCAF, MIP-1-alpha, -beta, NAP-1 und IL-8.

Eine Reportergruppe ist ein Atom, Molekül oder eine Verbindung, welche in Verbindung mit einem hierauf abgestellten Assay den Nachweis der Reportergruppe und der somit mit der Reportergruppe verbundenen Verbindung oder Substanz ermöglicht. Beispiele für Reportergruppen und hiermit assoziierte Detektionsmethoden sind: 32P-Labeling und Intensitätsmessung mittels Phosphoimager. Viele weitere Beispiele sind dem Durchschnittsfachmann bekannt und bedürfen nicht der detaillierten Aufzählung.

Eine an ein Targetmolekül bindende Substanz kann eine Substanz sein, welche ein Targetmolekül-Protein, eine Targetmolekül-RNA, oder eine Targetmolekül-DNA bindet. Mit umfasst sind dabei bezüglich der DNA auch Inhibitoren Transkription der Targetmolekül DNA und/oder Inhibtoren der die Targetmolekül-Genexpression regulierenden Region.

Im Rahmen der vorstehenden Definition gegenüber dem engen Wortsinn erweiterte Begriffsbestimmungen umfassen auch die bestimmten Begriffe im engen Wortsinn.

Vorbekannte Sequenzen und/oder deren Verwendung, welche unter die im Rahmen dieser Beschreibung verwendete Definitionen fallen, können durch einen Disclaimer in Patentansprüchen ausgeschlossen werden.

### Beispiele.

Im Folgenden wird die Erfindung anhand von lediglich bevorzugte Ausführungsformen darstellenden Beispielen und Figuren näher erläutert. Dabei ist Gegenstand insbesondere der Subtyp des Bronchialkarzinoms. Es zeigen:
- Figur 1:: Chip-Analysen zur differenziellen Expression der 489 Gene in Lungentumorgewebe aus 26 Patienten (14 Patienten mit Plattenepithelkarzinom und 12 Patienten mit Adenokarzinom). Aufgetragen auf der Abzisse sind einzelne Chipexperimente von entweder Tumor- oder Normalgewebe. Die linke Ordinate stellt die relative Expression (PMQ) des Gens SeqID162 / SeqID651 dar, während rechts die Signifikanz (p-Val) aufgetragen ist,
- Figur 2:: Immunhistochemische Anfärbung der 489 Gene bzw. deren Proteine mittels spezifischer Antik÷rper (z.B.: SeqID162 / SeqID651),

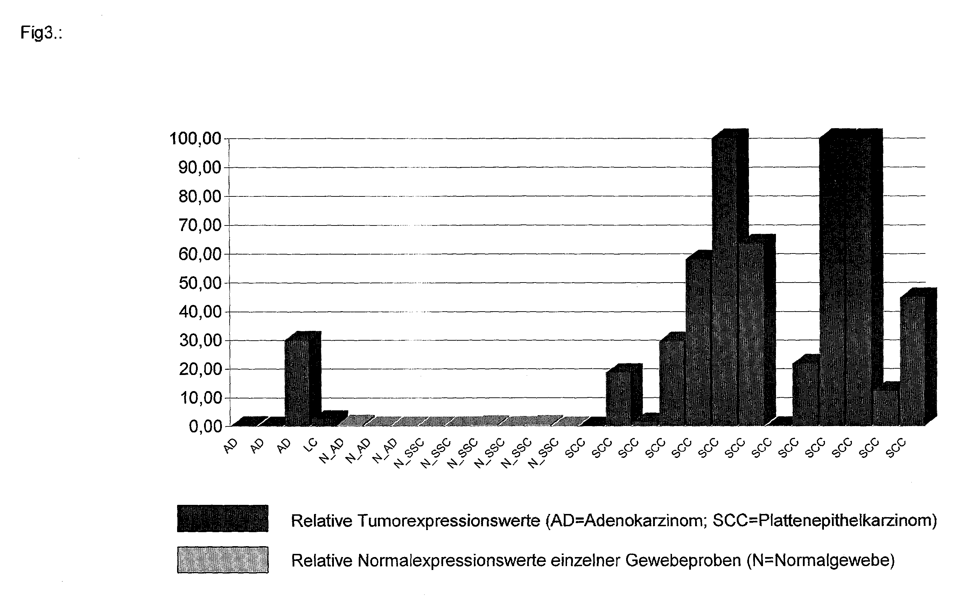

- Figur 3:: Taqman-Analyse zur differentiellen Expression von 489 Genen im Lungentumorgewebe (nach erfolgter Laser-gestützter Mikrodissektion der Normalepithelien und Tumorzellen) (z.B.: SeqID162 / SeqID651),
- Figur 4:: Taqman-Analyse zur spezifischen Expressionsverteilung von 489 Genen in 26 human Normalgeweben (z.B.: SeqID162 / SeqID651),
- Figur 5:: Taqman-Analyse zur differentiellen Expression von 489 Genen im Lungentumorgewebe (bulk), insbesondere im Subtyp des Plattenepithelkarzinoms, aus 11 Patienten (z.B.: SeqID162 / SeqID651),
- Figur 6:: Northern-Blot-Verfahren des sogenannten Cancer-Profiling-Arrays zur differentiellen Expression von 489 Genen insbesondere im Brochial-, Kolon-, Mamma-, Uterus- bzw. Endometrium-, Ovarial- und Rektalkarzinomgewebe (z.B.: SeqID162 / SeqID651).
- Figur 7:: Northern-Blot-Verfahren zur sogenannten Multiple-Tissue-Analyse zur spezifischen Expression von 489 Genen in 16 Normalgeweben eines Patienten (z.B.: SeqID162 / SeqID651).
- Figur 8:: Western Blot Analyse zur Überprüfung der erhöhten Proteinexpression in Bronchialtumorgeweben von 489 Antiseren, welche spezifische ihre 489 Antigene (Proteine) detektieren (z.B.: SeqID162 / SeqID651).

Enstprechende Experimente für andere Gene bzw. Sequenzen sind lediglich der Übersichtlichkeit halber nicht dargestellt.

### Beispiel 1: Mikrodissektion

Bronchialtumor- und -normalgewebe aus jeweils einem Patienten wurde gefroren und in 5 µm Proben geschnitten. Aus jedem Patienten wurden zumindest 30 Proben gewonnen. Normal und maligne Bereiche wurden durch einen Pathologen mit Hilfe eines Mikroskopes identifiziert und markiert. Die jeweiligen Bereiche wurden unter dem Mikroskop resektiert unter Verwendung eines computergestützten Lasers und jeweils separat auf -80°C eingefroren in 150 µl GTC-Puffer enthaltend 2% ß-Mercaptoethanol.

### Beispiel 2: Chipanalyse

Aus Proben aus Beispiel 1 wird RNA isoliert, amplifiziert und markiert. Hierzu wird folgende Technik verwendet.

Die Präparation von Poly-A+-RNA erfolgt unter Verwendung eines modifizierten Protokolls gemäß dem Poly-A-Tract 1000 Kit (Promega, Deutschland). Gewebeproben werden langsam auf Eis aufgetaut, zerkleinert und mit 300µl Verdünnungspuffer, enthaltend 1% ß-Mercaptoethanol, sowie biotinyliertem Oligo-dT Primer versetzt, und für 5 min. auf 70°C erhitzt. Die Proben werden dann für 5 min. bei 20°C gehalten und anschließend bei 20000g für 10 min. zentrifugiert. Dem Überstand werden 120µl gewaschener Streptavidin-gekoppelter paramagnetischer Partikel (SA-PMP) zugebenen und es wurde bei 20°C für 5 min. inkubiert. Die mRNA wurde dann durch magnetische Trennung isoliert. Nach drei Waschschritten mit 0,5x SSC Lösung wird die mRNA in Nuklease-freiem Wasser verdünnt, eingedampft unter Vakuum und umgehend wie folgt in cDNA prozessiert.

Die mRNA wird in 10µl Nuklease-freiem Wasser gelöst. 1µl T7-dT24-(GGCCAG) Primer (100 pmol/µl) wird zugegeben und es wird auf 70°C für 5 min. erhitzt. Dann wird die Probe auf Eis gelegt und es werden 4µl 5x first strand buffer (Invitrogen), 2µl DTT (0,1M), 1µl dNTP's (10mM), und 14U anti-RNAse (Ambion) zugegeben, gefolgt von einer Inkubation für 2 min. bei 37°C. Dann werden 1µl Superscript II Reverse Transskriptase (Invitrogen) zugegeben, gefolgt von einer Inkubation für 1 h bei 37°C.

Anschließend erfolgt die Zweitstrangsynthese und DNA Reinigung. Sofort nach der Synthese des ersten Stranges, wie vorstehend, werden 91µl Wasser, 30µl 5x second strand buffer, 3µl dNTP's (10mM), 10U E. coli DNA-ligase, 40U DNA Polymerase I und 2U RNAse H (alle von Invitrogen) zugegeben und die Mischung wird für 2 h bei 16°C inkubiert. Dann werden 10U T4 DNA Polymerase (Invitrogen) zugegeben und weitere 5 min. inkubiert. Die Reaktion wird durch Zugabe von 10µl 0,5mM EDTA abgebrochen. Die Reinigung der DNA erfolgt gemäß den Vorschriften des GFX PCR DNA and Gel Band Purification Kits (Amersham). Gereinigte DNA wird unter Vakuum eingedampft und bei -20°C gelagert.

Dann erfolgt die in vitro Transkription und cRNA Reinigung. Die in vitro Transskription wird gemäß dem Herstellerprotokoll von Ambion (Huntigdon, UK) durchgeführt. Das DNA Pellet wird in 8µl Wasser gelöst und 7,5µl dNTP's (75mM), 2µl 10x reaction buffer (Ambion), 2µl 10 T7 Enzymmix (Ambion) und 14U anti-RNAse (Ambion) werden zugegeben, gefolgt von einer Inkubation von 6 h bei 37°C. Die Reinigung der erhaltenen cRNA erfolgt gemäß dem Herstellerprotokoll zum Rneasy Mini Kit (Qiagen, Hilden, Deutschland). Nach Elution von der Säule wird die verdünnte cRNA eingedampft unter Vakuum und auf -80°C eingefroren.

Anschließend wird eine zweite und dritte in vitro Transskriptionsrunde durchgeführt. Diese Verstärkungsrunden werden mit nur geringen Abweichungen von der ersten Runde durchgeführt. Die Synthese des ersten Stranges erfolgt mit Random Hexamer Primern (250ng/µl). Nach Inkubation bei 37 °C für 60 min. wird das cRNA-cDNA Hybrid für 20 min. mit 2U RNase H bei 37 °C inkubiert, gefolgt von einem 2-minütigen Inaktivierungsschritt bei 37°C. Die Zweitstrang-Synthese wird in dieser zweiten und dritten Runde wie oben für die erste Runde beschrieben durchgeführt. Die in vitro Transkription wird in der dritten Runde mit biotinmarkierten NTP's durchgeführt.

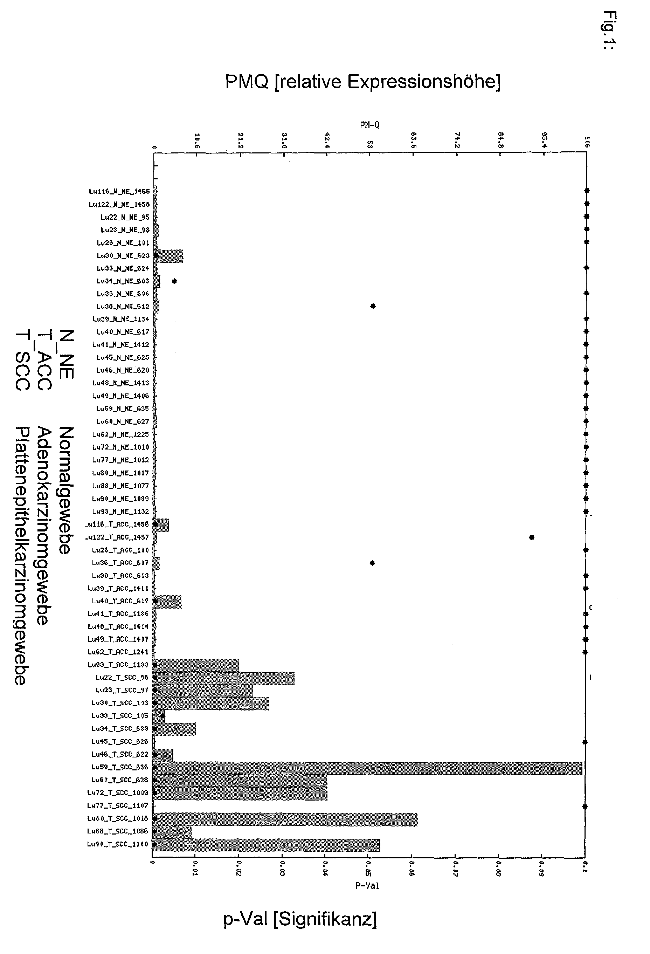

Die so erhaltene biotinylierte RNA wird einem Genchip aufgegeben, welcher eine Vielzahl von verschiedenen Oligonukleotiden enthält, wobei jeweils eines (oder auch mehrere, zu Kontrollzwecken) für ein definiertes Gen repräsentativ ist, i.e. eine charakteristische Teilsequenz hieraus aufweist. Man erhält sowohl qualitative, wie auch quantitative Information, ob eine betreffende Normalund/oder Tumorprobe ein betreffendes Gen exprimiert, und zwar auch im Verhältnis Tumor/Normal. In Fällen, in welchen ein Gen in Tumorgewebe höher exprimiert ist, als im korrelierten Normalgewebe liegt diffentielle Expression vor, i.e. das Gen ist im Tumorgewebe hochreguliert. Wenn das Gen dagegen in Tumorgewebe geringer exprimiert ist, liegt Herunterregulation vor. Es wurde gefunden, dass die analysierten erfindungsgemäßen Sequenzen differenziell im Tumorgewebe reguliert sind (Fig. 1).

### Beispiel 3: Untersuchung der Expression bzw. Überexpression mittels quantitativer PCR.

Die differenzielle Expression der Targetgene wird mittels quantitativer Echtzeit-PCR (TaqMan System) untersucht. Dazu wird die RNA, beispielsweise aber nicht notwendigerweise erhalten gemäß Beispiel 2 (cDNA nach der 1. in vitro Transkription), verwendet. 1ng cDNA werden für die Amplifikation eingesetzt mit 2,5µl 10x SYBR®Green PCR Puffer, 3µl Magnesiumchlorid (25mM), 2µl dNTP's (mit dUTP; 12,5 mM) und 0,625U Ampli Taq Gold in einem Reaktionsvolumen von 25µl. Die Reaktion wird in einem GeneAmp 5700 Sequence Detection System (Applied Biosystems, Weiterstadt, Deutschland) durchgeführt. Die Bedingungen sind: 2 min. 50°C, 10 min. 95°C, 15 s 95°C, 1 min. 60°C, die letzten beiden Phasen in 40 Zyklen. Für die jeweiligen Gene werden die geeigneten Vorwärts- bzw. Rückwärtsprimer verwendet. Die Auswertung erfolgt nach der ΔΔCt Methode nach Herstellervorschrift. Der Ct Wert von beta actin wurde bei einer Grenze von 0,1 gemessen. Zur Normalisierung wird der Ct Wert des beta actin vom Ct Wert des untersuchten Gens abgezogen. Dieser normalisierte Ct Wert wird im Falle der Tumorgewebe auf die Normalgewebe bezogen bzw. normalisiert, wodurch der ΔΔCt erhalten wird.

Wird dieser Wert als Potenz zur Basis 2 eingesetzt, so wird eine relative Größe der Über- oder Unterexpression in Tumorgewebe gegenüber dem Normalgewebe des gleichen Patienten erhalten. Im Ergebnis kann so bestimmt werden, ob ein spezifisches Tumorgewebe eines bestimmten Patienten sensitiv für eine erfindungsgemäße Behandlung ist. Auch kann mit dieser Methode bestimmt werden, ob nicht klassifiziertes Gewebe als Tumorzellen enthaltend einzustufen ist. In letzterem Falle erfolgt ein Vergleich zu Referenzwerten bzw. klassifiziertem Normalgewebe des gleichen Patienten oder von anderen Personen.

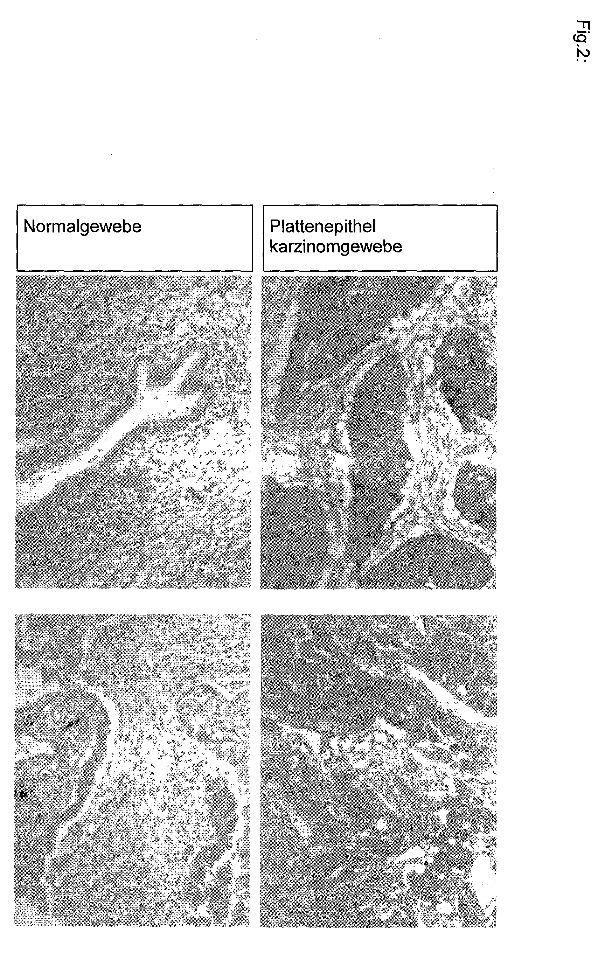

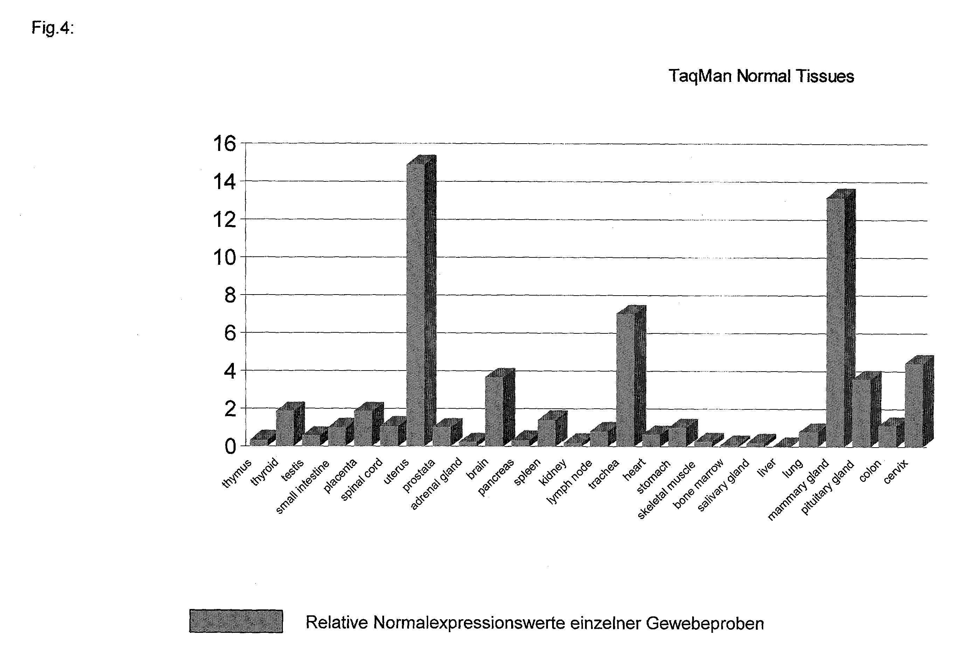

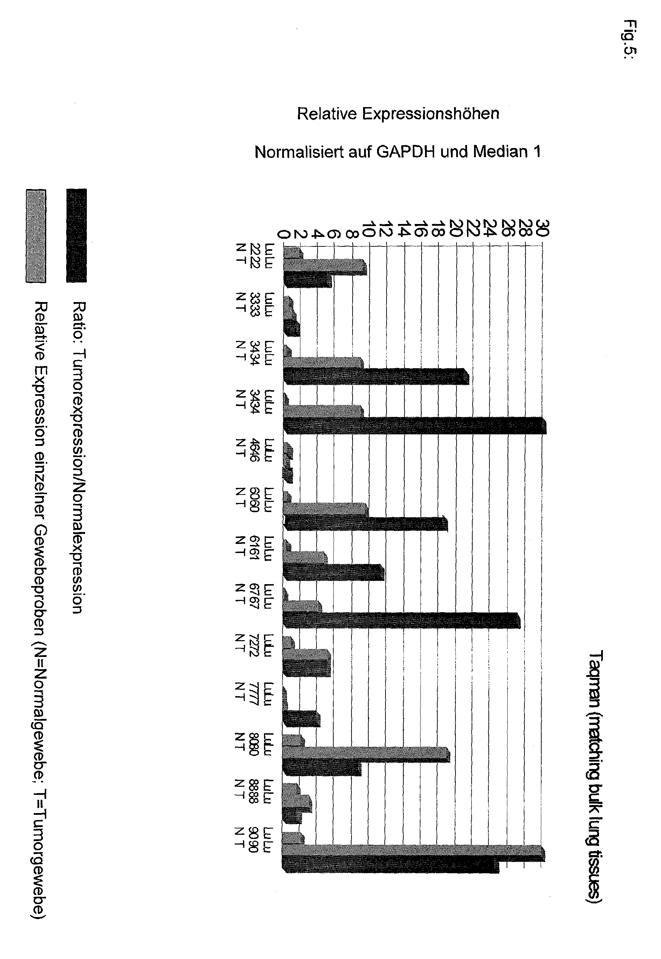

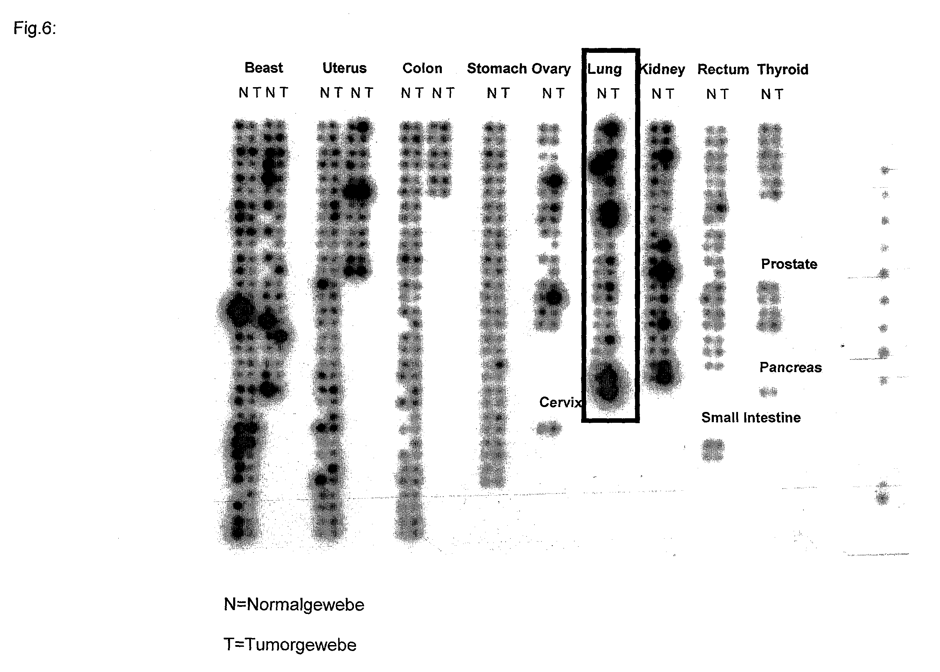

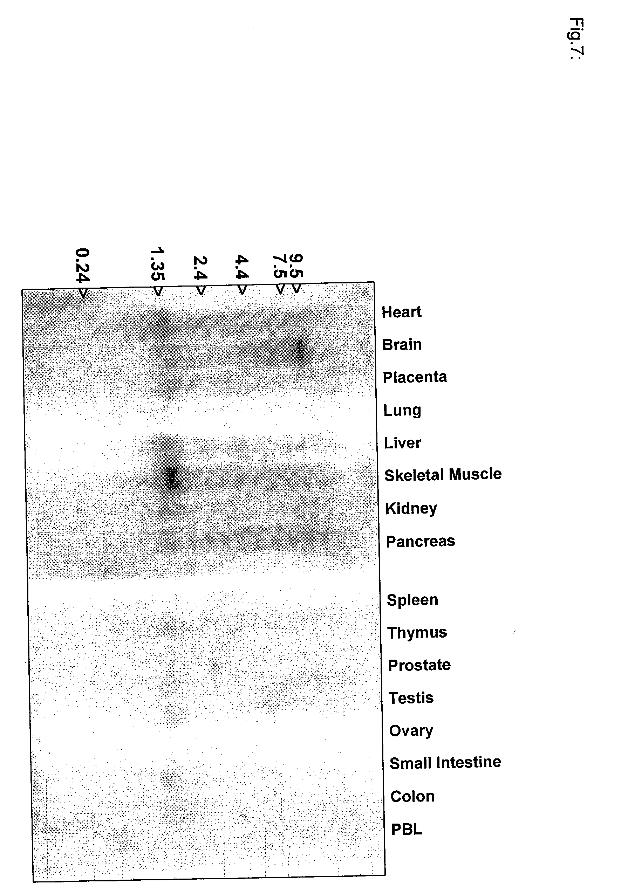

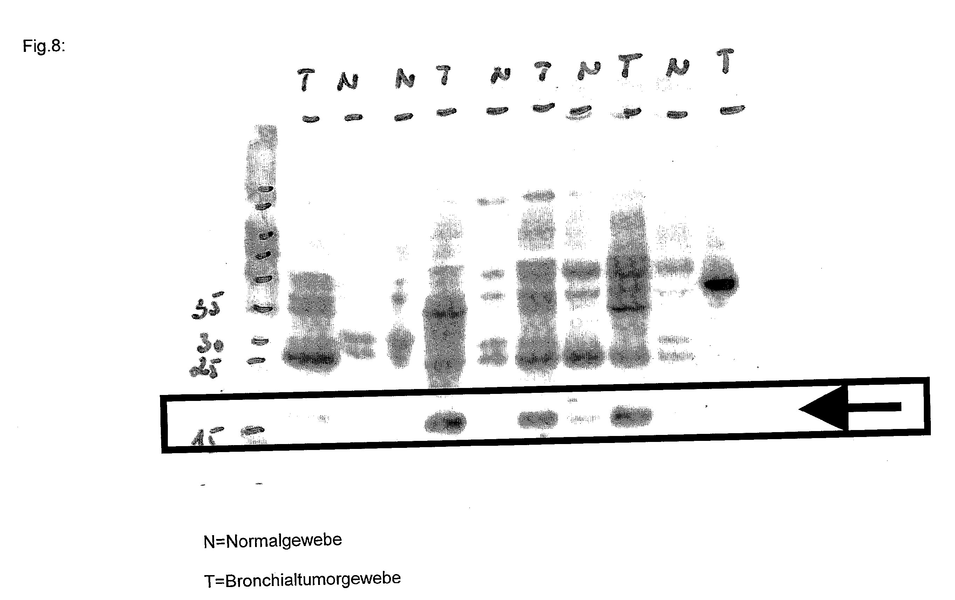

Ergebnisse für die Überexpression des Gens Seq.-ID 162 bzw. 651 in Bronchialtumoren sind der Figur 2 zu entnehmen. In der Figur 4 erkennt man, dass die Expression in verschiedenen Normalgeweben sehr unterschiedlich, teilweise auch sehr hoch ist. In der Lunge wird ein nur sehr geringes Expressionsniveau festegestellt. In der Figur 5 erkennt man schließlich, dass das untersuchte Gen insbesondere im Subtyp des Plattenepithelgewebes differenziell exprimiert wird. Entsprechende Ergebnisse werden mittels Northern Blots (Figuren 6 und 7) und Western Blots (Figur 8) erhalten, wodurch auch auf Proteinebene eine Verifizierung der Ergebnisse auf Nukleinsäurenebene erfolgt.

### Beispiel 4: Nachweis eines überexprimierten Gens mittels Antikörpern.

In diesem Beispiel wird die Markierung von Tumorzellen durch einen gegen ein erfindungsgemäßes Protein gerichteten Antikörper in vivo (Mausmodell) beschrieben. Ein solcher erfindungsgemäßer Antikörper wird mit einem Markermolekül (z.B. Radioisotop) markiert. In NMRI-Nacktmäuse werden mit einem erfindungsgemäßen Gen transfizierte humane Zellen transplantiert. Nach einem definierten Zeitraum, beispielsweise 30 Tage, wird den Mäusen der markierte Antikörper injiziert. Die Kontrolltiere werden mit einem nicht relevanten Antikörper behandelt. Wenige Stunden nach der Antikörperapplikation werden die Tiere getötet und aus allen Organen Gewebeschnitte angefertigt. Diese Schnitte werden auf die Gegenwart von markiertem Antikörper untersucht.

Bei den Antikörpern kann es sich im einfachsten Fall um polyklonale Antikörper gegen humanes Protein, konjugiert mit einem Trägerprotein, in Kaninchen gezogen und mit den spezifischen immobilisierten Peptiden affinitätsgereinigt, handeln. Geeignete Immunisierungspeptide sind beispielsweise aus Teilsequenzen eines erfindungsgemäßen Proteins gebildet. Als Immunogene können ebenso mit cDNA des Gens, oder Teilsequenzen hiervon transfizierte Zellen, wie beispielsweise COS-Zellen oder NIH3T3-Zellen, eingesetzt werden. Ebenso sind Tumorzellen, die endogen das Protein exprimieren, geeignet. Weiterhin kann auch rekombinant hergestelltes Protein bzw. Teilsequenzen hieraus, die in Producerzellen, wie E. coli oder Eukaryontenzellen, wie Insektenzellen, exprimiert werden, zur Immunisierung eingesetzt werden. Selbstverständlich können stattdessen auch entsprechende monoklonale Antikörper oder Fragmente hiervon eingesetzt werden.

Das hier untersuchte Gen zeigt gemäß der Figur 2 eine vergleichsweise starke Färbung in Plattenepithelkarzinomgewebe.

### Beispiel 5: Immunhistochemischer Nachweis von Tumorzellen.

Gewebe wird aus einem Patienten mit Krebs oder dem Verdacht auf Krebs isoliert und als Paraffin- bzw. Gefrierschnitte präpariert. Diese Schnitte werden mit einem gegen ein erfindungsgemäßes Protein gerichteten Antikörper auf die Überexpression des Proteins in Tumorzellen untersucht. Die immunhistologische Untersuchung mit dem Antikörper zeigt höhere Expression des Proteins in den Tumorzellen im Vergleich zu umliegenden Normalgewebe. Die Untersuchung erfolgt im Einzelnen durch Inkubation mit dem Antikörper als primärem Antikörper, einem biotinyliertem sekundären anti-Kaninchen Antikörper und einer Streptavidin-gekoppelten Meerrettichperoxidase. Die Färbung erfolgt mit mit DAB als chromogenen Substrat (braune Färbung). Die Gegenfärbung erfolgt mit Hemalaun-Lösung (blaue Färbung). Es sind maligne und nichtmaligne Zellen unterscheidbar, wobei die malignen Zellen eine starke Färbung, i.e. hohen Gehalt an erfindungsgemäßem Protein, aufweisen, während die nichtmalignen Zellen nur moderat gefärbt sind.

Im Falle einer Erkrankung erkennt man im Falle der Überexpression in Tumorgewebe eine vergleichsweise starke Färbung.

### Beispiel 6: Erzeugung von anti-idiotypischen monoklonalen Antikörpern zu therapeutischen Zwecken

Ausgehend von einem erfindungsgemäßen Protein wird in fachüblicher Weise ein monoklonaler Antikörper Ab1 erzeugt, welcher in der Lage ist, das beispielsweise das Protein Seq.-ID 651 spezifisch zu erkennen und daran zu binden. Dabei ist es unwesentlich, ob eine funktionale Domäne oder ein anderer zugänglicher Bereich erkannt wird. Mit Hilfe des erzeugten Antikörpers Ab1 wird in ebenso fachüblicher Weise ein zweiter anti-idiotypischer nicht humanisierter, beispielsweise Maus, monoklonaler Antikörper aAB1 erzeugt, welcher zur Herstellung einer pharmazeutischen Zusammensetzung zur Behandlung von Bronchialtumoren geeignet ist. Die Funktion des Antikörpers aAB1 beruht dabei darauf, dass dieser dem humanen Immunsystem ein Image des (humanen) Protein-Antigens gleichsam vortäuscht, wobei das Immunsystem den Antikörper aAB1 aufgrund seiner mangelnden Humanisierung als körperfremd erkennt. Der humane Körper bildet folglich eigene Antikörper, die gegen aAB1 und somit auch gegen das humane Protein bzw. dieses exprimierende Tumorzellen gerichtet sind.

## Patentansprüche

1. Nukleinsäure, insbesondere isolierte Nukleinsäure, enthaltend oder bestehend aus einer Nukleinsäuresequenz gemäß einer der Sequenzen Seq.-ID 1 bis 489.

2. Peptid oder Protein, insbesondere isoliertes Peptid oder Protein, enthaltend eine Aminosäurensequenz codiert durch eine der Nukleinsäuresequenzen Seq.-ID 1 bis 489 oder bestehend hieraus oder enthaltend eine Aminosäuresequenz gemäß einer der Sequenzen Seq.-ID 490 bis 978 oder bestehend hieraus.

3. Verwendung einer Nukleinsäure und/oder eines Peptids oder Proteins nach Anspruch 1 oder 2, zur Detektion von Bronchialkrebs oder zur Detektion eines Risikos der Erkrankung an Bronchialkrebs, wobei eine Bronchial-Gewebeprobe auf differenzielle Transkription der Nukleinsäure oder auf differenzielle Expression des Proteins untersucht wird und/oder wobei die Nukleinsäure und/oder das Peptid oder Protein in Blutserum oder Sputum detektiert wird.

4. Verwendung nach Anspruch 3, wobei eine an die Nukleinsäure oder eine an das Protein oder Peptid bindende Detektorsubstanz, vorzugsweise enthaltend eine Reportergruppe, verwendet wird, wobei Bindung besagter Nukleinsäure und/oder besagten Proteins oder Peptids an die Detektorsubstanz halbquantitativ oder quantitativ detektiert wird.

5. Verwendung einer Nukleinsäure oder eines Proteins oder Peptids nach Anspruch 1 oder 2, zum Screenen nach daran bindenden Substanzen, insbesondere prospektiven Wirkstoffen zur Inhibierung von besagter Nukleinsäure oder besagtem Protein oder Peptid, oder prospektiven Detektorsubstanzen, wobei eine prospektive Substanz oder eine Mischung solcher prospektiver Substanzen mit besagter Nukleinsäure oder besagtem Protein oder Peptid kontaktiert wird, wobei mit einem Bindungsassay Bindungsereignisse festgestellt werden, und wobei eine bindende prospektive Substanz, ggf. nach Dekonvolution, selektiert wird.

6. Verwendung einer eine Nukleinsäure oder ein Peptid bzw. Protein nach Anspruch 1 oder 2 inhibierenden oder daran bindenden Substanz, insbesondere identifiziert nach Anspruch 5, zur Herstellung einer pharmazeutischen Zusammensetzung zur Diagnose und/oder Behandlung von Bronchialkrebs.

7. Verwendung nach Anspruch 6, wobei die Substanz ausgewählt ist aus der Gruppe bestehend aus:
a) Antisense-Oligonukleotide, siRNA, und Ribozyme gegen eine Nukleinsäure nach Anspruch 1,
b) an ein Peptid oder Protein nach Anspruch 2 bindendes, insbesondere nach Anspruch 5 identifiziertes, organisches Molekül mit einem Molekulargewicht unterhalb 5000, vorzugsweise unterhalb 1000, höchstvorzugsweise unterhalb 300,
c) Aptamer gegen ein Protein oder Peptid nach Anspruch 2, insbesondere identifiziert nach Anspruch 5,
d) (monoklonaler) Antikörper, insbesondere humaner oder humanisierter Antikörper, gegen ein Protein oder Peptid nach Anspruch 2,
e) anti-idiotypische nicht-humane (monoklonale) Antikörper, generiert mittels eines Antikörpers der Unterguppe d), und
f) vorstehende Substanzen derivatisiert mit einer Reportergruppe, einem Zelltoxin einer immunstimulierenden Komponente und/oder einem Radioisotop.

8. Verwendung nach einem der Ansprüche 6 oder 7, wobei die pharmazeutische Zusammensetzung zur lokalen Applikation in Tumorzellen enthaltendem Gewebe hergerichtet ist.

9. Verfahren zur Diagnose einer Bronchialkrebserkrankung, wobei eine pharmazeutische Zusammensetzung nach einem der Ansprüche 6 bis 8 in der Ausführungsform mit einer Reportergruppe in zu untersuchendes Gewebe in vivo oder in vitro appliziert wird, wobei das zu untersuchende Gewebe dann einer Detektionsverfahrenstufe unterworfen wird, welche sensitiv für die Reportergruppe ist, und wobei im Fall der Detektion eines definierten Mindestwertes (Überexpression in Tumorgewebe) oder eines definierten Maximalwertes (Unterexpression in Tumorgewebe) der Reportergruppe im Gewebe optional das Gewebe als Tumorzellen enthaltend qualifiziert wird, oder wobei eine pharmazeutische Zusammensetzung nach einem der Ansprüche 6 bis 8 in der Ausführungsform mit einer Reportergruppe mit einer Blutserumprobe und/oder einer Sputumprobe kontaktiert wird, wobei die Probe dann einer Detektionsverfahrenstufe unterworfen wird, welche sensitiv für die Reportergruppe ist, und wobei im Fall der Detektion eines definierten Mindestwertes oder eines definierten Maximalwertes der Reportergruppe in der Probe optional Bronchialgewebe des zugeordneten Patienten als Tumorzellen enthaltend qualifiziert wird.

10. Verfahren zur Behandlung einer Bronchialtumor-Erkrankung, wobei eine pharmazeutische Zusammensetzung nach einem der Ansprüche 6 bis 8 in einer physiologisch wirksamen Dosis einem Patienten dargereicht wird.
